# EUROPEAN PATENT APPLICATION

(11) **EP 4 201 393 A1**
(43) Date of publication of application: **28.06.2023**
(21) Application number: 21858088.4
(22) Date of filing: 15.07.2021
(51) Int. Cl.: A61K 8/31, A61K 8/19, A61K 8/34, A61K 8/37, A61K 8/39, A61K 8/84, A61K 8/891, A61K 8/92, A61Q 1/04

(54) **COMPOSITION FOR EXTERNAL PREPARATION FOR SKIN**

(30) Priority: 21.08.2020 JP 2020140118
(71) Applicant: Shiseido Company, Ltd., Chuo-ku Tokyo 104-0061 (JP)
(72) Inventor: NAKATA Hiroko, Tokyo 104-0061 (JP); KOSAKA Kyohei, Tokyo 104-0061 (JP)
(74) Representative: Ter Meer Steinmeister & Partner
(86) International application number: PCT/JP2021/026690
(87) International publication number: WO 2022/038939

(57) **Abstract**

An external-use skin preparation composition contains (A) from 15 % by mass to 40 % by mass of microcrystalline wax, (B) a solid-form oily gelling agent including at least one selected from the group consisting of glyceryl behenate/eicosadioate, hydrogenated vegetable oil, jojoba ester, behenyl alcohol, stearyl alcohol, castor oil/IPID copolymer, caprylic/capric triglyceride, glyceryl behenate, and polyglyceryl-6 octastearate, and (C) from 2 % by mass to 75 % by mass of a liquid-state oily component. Component (C) is a liquid-state oily component wherein a mixture obtained by melt-mixing component (A) and component (C) at a mass ratio of 1:3 and then solidifying the same has a 3-mm penetration hardness (needle diameter: 5.6 ϕ) of 30 or greater at 25°C.

## Description

### Cross Reference to Related Applications

The present application is based upon and claims the benefit of priority from Japanese Patent Application No. 2020-140118 filed on August 21, 2020, the entire contents of which are incorporated herein by reference.

### Technical Field

The present disclosure relates to an external-use skin preparation composition. Particularly, the present disclosure relates to an oily external-use skin preparation composition.

### Background Art

There are cases in which it is desirable that a region coated with an external-use skin preparation, such as a moisturizer, cosmetic, etc., does not have luster (gloss). For example, Patent Literature 1 discloses a lusterless lip rouge cosmetic.

The stick-form lip rouge cosmetic disclosed in Patent Literature 1 includes 20 to 50 wt% of powder and 20 to 50 wt% of an open-chain dimethyl polysiloxane having a viscosity of 5 to 8 cs, wherein candelilla wax is used as a polar wax, 25 to 30 wt% of kaolin is included as an extender pigment, and 15 wt% of wax is included.

### Citation List

### Patent Literature

Patent Literature 1: Japanese Examined Patent Publication No. H07-20850

### Summary of Invention

### Technical Problem

The following analysis can be made from the perspective of the present disclosure.

There are also cases in which it is desirable that an external-use skin preparation can be applied thickly to the skin, in addition to not having luster. For example, in cases of moisturizers, moisturizing effects can be further improved if the preparation can be applied thickly. In cases of cosmetics, makeup lastability can be improved if the preparation can be applied thickly and uniformly. In order to be able to apply an external-use skin preparation thickly and uniformly, the external-use skin preparation is required to be soft to some extent and also smooth. Unfortunately, a coating film made from a soft external-use skin preparation tends to exhibit luster, and it is not possible to achieve a matte finish. On the other hand, generally speaking, if a preparation has a compositional makeup to prevent luster (i.e., to achieve a matte finish) as in the stick-form cosmetic disclosed in Patent Literature 1, the external-use skin preparation tends to become hard and unsmooth, making it impossible to apply the preparation thickly and uniformly.

Hence, there is a demand for an external-use skin preparation composition with which a coating film can be formed thickly and uniformly and with which the coating region can be provided with a matte finish.

According to a first aspect of the present disclosure, an external-use skin preparation composition is provided, the composition comprising (A) from 15 % by mass to 40 % by mass of microcrystalline wax; (B) a solid-form oily gelling agent including at least one selected from the group consisting of glyceryl behenate/eicosadioate, hydrogenated vegetable oil, jojoba ester, behenyl alcohol, stearyl alcohol, castor oil/IPID copolymer, caprylic/capric triglyceride, glyceryl behenate, and polyglyceryl-6 octastearate; and (C) from 2 % by mass to 75 % by mass of a liquid-state oily component. The component (C) is a liquid-state oily component wherein a mixture obtained by melt-mixing the component (A) and the component (C) at a mass ratio of 1:3 and then solidifying the same has a 3-mm penetration hardness (needle diameter: 5.6 ϕ) of 30 or greater at 25°C.

According to a second aspect of the present disclosure, an external-use skin preparation composition is provided, the composition comprising (A) from 20 % by mass to 40 % by mass of microcrystalline wax; (B) a solid-form oily gelling agent including at least one selected from the group consisting of glyceryl behenate/eicosadioate, hydrogenated vegetable oil, jojoba ester, behenyl alcohol, stearyl alcohol, castor oil/IPID copolymer, caprylic/capric triglyceride, glyceryl behenate, and polyglyceryl-6 octastearate; (D) from 30 % by mass to 50 % by mass of a liquid-state hydrocarbon oil; and (E) Vaseline. A content of the component (E) is 0.8 parts by mass or less relative to 1 part by mass of the component (A).

### Advantageous Effects of Invention

The external-use skin preparation composition of the present disclosure can be applied thickly with a uniform thickness and can achieve a matte finish in the coating region.

### Description of Embodiments

Preferred modes according to the aforementioned aspects will be described below.

According to a prefered mode of the above first aspect, the component (C) includes at least one selected from the group consisting of liquid-state ester oils, liquid-state higher alcohols, and liquid-state silicone oils.

According to a prefered mode of the above first aspect, the external-use skin preparation composition further comprises (D) from 5 % by mass to 70 % by mass of a liquid-state hydrocarbon oil.

According to a prefered mode of the above first aspect, a content of (E) vaseline is 3 % by mass or less relative to the mass of the external-use skin preparation composition.

According to a prefered mode of the above second aspect, the content of the component (E) is from 5 % by mass to 30 % by mass relative to the mass of the external-use skin preparation composition.

According to a prefered mode of the above second aspect, the external-use skin preparation composition further comprises (C) from 5 % by mass to 30 % by mass of a liquid-state oily component. The component (C) is a liquid-state oily component wherein a mixture obtained by melt-mixing the component (A) and the component (C) at a mass ratio of 1:3 and then solidifying the same has a 3-mm penetration hardness (needle diameter: 5.6 ϕ) of 30 or greater at 25°C.

According to a prefered mode of the above second aspect, the external-use skin preparation composition, the component (C) includes from 5 % by mass to 30 % by mass of a liquid-state oily component, including at least one selected from the group consisting of liquid-state ester oils, liquid-state higher alcohols, and liquid-state silicone oils.

According to a prefered mode of the above first and second aspects, the external-use skin preparation composition, a content of the component (B) is from 0.0015 to 0.5 parts by mass relative to 1 part by mass of the component (A).

According to a prefered mode of the above first and second aspects, the external-use skin preparation composition further comprises (F) from 0.1 % by mass to 15 % by mass of a powder.

According to a prefered mode of the above first and second aspects, a content of water is 5 % by mass or less relative to the mass of the external-use skin preparation composition.

According to a prefered mode of the above first and second aspects, a hardness measured under the following conditions is from 30 to 100: load: 2 kg; needle diameter: 5.6 ϕ; penetration depth: 3 mm; rising speed: 20 mm/min.; measurement temperature: 25°C.

According to a prefered mode of the above first and second aspects, the external-use skin preparation composition has a cream form or a paste form.

According to a prefered mode of the above first and second aspects, the external-use skin preparation composition is applicable as a moisturizer.

According to a prefered mode of the above first and second aspects, the external-use skin preparation composition is applicable as a lip cosmetic.

In the following description, POE is an abbreviation of polyoxyethylene, and POP is an abbreviation of polyoxypropylene. The number in parentheses after POE or POP indicates the average number of moles of POE groups or POP groups added in the compound in question.

In the present disclosure, "substantial amount" refers to an amount capable of bringing about the actions/effects achieved by the addition of the compound in question.

In the present disclosure, "solid-form" refers to a form/state capable of maintaining a solid form under atmospheric pressure at 25°C. In the present disclosure, "liquid-state" refers to a form/state having flowability (i.e., is a liquid) under atmospheric pressure at 25°C.

### First Embodiment:

An external-use skin preparation composition according to a first embodiment of the present disclosure will be described. The external-use skin preparation composition according to the first embodiment includes (A) microcrystalline wax, (B) a solid-form oily gelling agent, and (C) a liquid-state oily component.

(A) Microcrystalline Wax:
   In the first embodiment, the content of the component (A) relative to the mass of the external-use skin preparation composition is preferably 15 % by mass or greater, more preferably 20 % by mass or greater. The content of the component (A) relative to the mass of the external-use skin preparation composition may be 25 % by mass or greater, or 30 % by mass or greater. If the content of the component (A) is less than 15 % by mass, a coating region coated with the external-use skin preparation composition may exhibit luster, and also, it becomes difficult to apply the external-use skin preparation composition thickly with a uniform thickness. The content of the component (A) relative to the mass of the external-use skin preparation composition may be 40 % by mass or less, 35 % by mass or less, 30 % by mass or less, or 25 % by mass or less.
(B) Solid-Form Oily Gelling Agent:
   The component (B) is a component that, by being used in combination with the component (A), is capable of increasing hardness such that at least the liquid-state oily component can be applied thickly. The component (B) may be at least one selected from the group consisting of glyceryl behenate/eicosadioate, hydrogenated vegetable oil, jojoba ester, behenyl alcohol, stearyl alcohol, castor oil/IPID copolymer, caprylic/capric triglyceride, glyceryl behenate, and polyglyceryl-6 octastearate.

The content of the component (B) relative to 1 part by mass of the component (A) may be 0.0015 parts by mass or greater, 0.002 parts by mass or greater, 0.003 parts by mass or greater, 0.005 parts by mass or greater, 0.008 parts by mass or greater, 0.01 parts by mass or greater, 0.03 parts by mass or greater, 0.05 parts by mass or greater, 0.07 parts by mass or greater, 0.1 parts by mass or greater, 0.2 parts by mass or greater, or 0.3 parts by mass or greater. The content of the component (B) relative to 1 part by mass of the component (A) may be 0.5 parts by mass or less, 0.4 parts by mass or less, 0.3 parts by mass or less, 0.2 parts by mass or less, 0.1 parts by mass or less, 0.08 parts by mass or less, 0.05 parts by mass or less, or 0.03 parts by mass or less.

The content of the component (B) relative to the mass of the external-use skin preparation composition may be 0.05 % by mass or greater, 0.1 % by mass or greater, 0.2 % by mass or greater, 0.5 % by mass or greater, or 0.8 % by mass or greater. The content of the component (B) relative to the mass of the external-use skin preparation composition may be 20 % by mass or less, 15 % by mass or less, 10 % by mass or less, 5 % by mass or less, 2 % by mass or less, 1.5 % by mass or less, or 1 % by mass or less.

### (C) Liquid-State Oily Component:

The component (C) is a component that, by being mixed with the component (A), enables preparation of a mixture having a moderate hardness as an external-use skin preparation, compared to the hardness of the component (A) used alone. The hardness of a mixture of the component (A) and the component (C) can be found as follows.

The component (A) and the component (C) are mixed at the following mass ratio, i.e., Component (A): Component (C) = 1:3. The mixture is heated and molten, is then mixed, and thereafter cooled to room temperature and solidified, to prepare a mixture in which the component (A) and the component (C) are mixed uniformly. The hardness of the mixture is measured under the following conditions: load: 2 kg; penetration depth: 3 mm; rising speed: 20 mm/min.; measurement temperature: 25°C. In cases where the needle diameter is 1 ϕ (diameter: 1 mm), the hardness of the mixture is preferably 1 or greater, more preferably 3 or greater, even more preferably 5 or greater. In cases where the needle diameter is 1 ϕ, the hardness of the mixture is preferably 200 or less, more preferably 170 or less. In cases where the needle diameter is 5.6 ϕ (diameter: 5.6 mm), the hardness of the mixture is preferably 30 or greater, more preferably 50 or greater, even more preferably 100 or greater.

It is preferred that the component (C) includes at least one selected from the group consisting of liquid-state ester oils, liquid-state higher alcohols, and liquid-state silicone oils. The component (C) of the present disclosure does not include liquid-state hydrocarbon oils.

Examples of the synthesis ester oils that may be used may include isopropyl myristate, cetyl octanoate, octyldodecyl myristate, isopropyl palmitate, butyl stearate, hexyl laurate, myristyl myristate, decyl oleate, hexyldecyl dimethyl octanoate, cetyl lactate, myristyl lactate, lanolin acetate, isocetyl stearate, isocetyl isostearate, cholesteryl 12-hydroxy stearate, ethylene glycol di-2-ethyl hexanoate, di-penta erythritol fatty acid ester, N-alkyl glycol monoisostearate, neopentyl glycol dicaprate, diisostearyl malate, glyceryl di-2-heptyl undecanoate, trimethyrol propane tri-2-ethyl hexanoate, trimethyrol propane triisostearate, pentaerythritol tetra-2-ethyl hexanoate, glyceryl tri-2-ethyl hexanoate, glyceryl trioctanoate, glyceryl triisopalmitate, trimethyrol propane triisostearate, cetyl 2-ethylhexanoate, 2-ethylhexyl palmitate, glyceryl trimyristate, glyceride tri-2-heptyl undecanoate, castor oil fatty acid methyl ester, oleyl oleate, acetoglyceride, 2-heptylundecyl palmitate, diisobutyl adipate, N-lauroyl-L-glutamic acid-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, triethyl citrate, and the like.

Preferable examples of liquid-state ester oils usable in the external-use skin preparation composition of the present disclosure include pentaerythrityl tetraethylhexanoate, neopentyl glycol dicaprate, diisostearyl malate, glyceryl diisostearate, caprylic/capric triglyceride, phytosteryl isostearyl dimer dilinoleate, ethylhexyl methoxycinnamate, polyglyceryl-2 triisostearate, polyglyceryl-2 diisostearate, sorbitan sesquiisostearate, etc.

Examples of the higher alcohol that may be used may include linear alcohol (such as lauryl alcohol, cetyl alcohol, stearyl alcohol, behenyl alcohol, myristyl alcohol, oleyl alcohol, and cetostearyl alcohol); branched-chain alcohol (such as monostearylglycerin ether (batyl alcohol), 2-decyltetradecinol, lanolin alcohol, cholesterol, phytosterol, hexyldodecanol, isostearyl alcohol, and octyldodecanol) and the like.

A preferable example of a liquid-state higher alcohol usable in the external-use skin preparation composition of the present disclosure includes octyldodecanol, etc.

Examples of the silicone oil may include silicone compounds such as dimethylpolysiloxane, methylhydrogenpolysiloxane, methylphenylpolysiloxane, stearoxymethylpolysiloxane, polyether-modified organopolysiloxane, fluoroalkyl/polyoxyalkylene co-modified organopolysiloxane, alkyl-modified organopolysiloxane, terminal-modified organopolysiloxane, fluorine-modified organopolysiloxane, amino-modified organopolysiloxane, silicone gel, acrylic silicone, trimethylsiloxysilicic acid, silicone RTV rubber and the like.

Preferable examples of liquid-state silicone oils usable in the external-use skin preparation composition of the present disclosure include volatile dimethicone (dimethyl polysiloxane), polysilicone-15, diphenyl dimethicone, decamethyl tetrasiloxane, trimethyl pentaphenyl trisiloxane, etc. A preferable example of volatile dimethicone may have a viscosity (25°C) of 2 cSt or less.

The viscosity of volatile dimethicone can be measured, for example, according to Kinematic Viscosity Measurement Methods-No. 1 "Capillary Viscometer Method" as stipulated in The Japanese Standards of Quasi-Drug Ingredients 2021.

The content of the component (C) relative to the mass of the external-use skin preparation composition may be 2 % by mass or greater, 5 % by mass or greater, 10 % by mass or greater, 20 % by mass or greater, 30 % by mass or greater, 40 % by mass or greater, or 50 % by mass or greater. The content of the component (C) relative to the mass of the external-use skin preparation composition may be 75 % by mass or less, 65 % by mass or less, 55 % by mass or less, 45 % by mass or less, 35 % by mass or less, 25 % by mass or less, or 15 % by mass or less.

### (D) Liquid-State Hydrocarbon Oil:

The external-use skin preparation composition according to the first embodiment may further include a liquid-state hydrocarbon. The component (D) as referred to in the present disclosure does not include microcrystalline wax and vaseline.

Examples of hydrocarbon oils may include liquid paraffin, ozokerite, squalane, pristane, paraffin, ceresin, squalene, isododecane, isohexadecane, hydrogenated polydecene, mineral oils, etc.

Preferable examples of liquid-state hydrocarbon oils usable in the external-use skin preparation composition of the present disclosure include hydrogenated polydecene, hydrogenated polyisobutene, etc.

The content of the component (D) relative to the mass of the external-use skin preparation composition may be 5 % by mass or greater, 10 % by mass or greater, 20 % by mass or greater, 30 % by mass or greater, 40 % by mass or greater, or 50 % by mass or greater. The content of the component (D) relative to the mass of the external-use skin preparation composition may be 70 % by mass or less, 60 % by mass or less, 50 % by mass or less, 40 % by mass or less, 30 % by mass or less, or 20 % by mass or less.

### (E) Vaseline:

In the external-use skin preparation composition according to the first embodiment, the content of the component (E) relative to the mass of the external-use skin preparation composition is preferably 3 % by mass or less, more preferably 2 % by mass or less, more preferably 1 % by mass or less. It is preferred that the external-use skin preparation composition according to the first embodiment substantially does not include vaseline. By reducing the content of vaseline, it is possible to improve the high-temperature stability of the external-use skin preparation composition.

### Second Embodiment:

Next, an external-use skin preparation composition according to a second embodiment of the present disclosure will be described. The external-use skin preparation composition according to the second embodiment includes (A) microcrystalline wax, (B) a solid-form oily gelling agent, (D) a liquid-state hydrocarbon oil, and (E) vaseline. The external-use skin preparation composition according to the first embodiment includes (C) a liquid-state oily component, but the external-use skin preparation composition according to the second embodiment does not necessarily have to include the component (C).

### (A) Microcrystalline Wax:

In the second embodiment, the content of the component (A) relative to the mass of the external-use skin preparation composition is preferably 20 % by mass or greater.

Modes of the component (A) of the second embodiment other than the above can be the same as those in the first embodiment. As regards the modes of the component (A) of the second embodiment other than the above, the description of the first embodiment is incorporated herein by reference, and description thereon is omitted herefrom.

### (B) Solid-Form Oily Gelling Agent:

Modes of the component (B) of the second embodiment can be the same as those in the first embodiment. As regards the modes of the component (B) of the second embodiment, the description of the first embodiment is incorporated herein by reference, and description thereon is omitted herefrom.

### (D) Liquid-State Hydrocarbon Oil:

In the second embodiment, the content of the component (D) relative to the mass of the external-use skin preparation composition may be 30 % by mass or greater, 35 % by mass or greater, or 40 % by mass or greater. The content of the component (D) relative to the mass of the external-use skin preparation composition may be 50 % by mass or less, 45 % by mass or less, or 40 % by mass or less.

Modes of the component (D) of the second embodiment other than the above can be the same as those in the first embodiment. As regards the modes of the component (D) of the second embodiment other than the above, the description of the first embodiment is incorporated herein by reference, and description thereon is omitted herefrom.

### (E) Vaseline:

In the external-use skin preparation composition according to the second embodiment, the content of the component (E) relative to 1 part by mass of the component (A) is preferably 0.8 parts by mass or less. The content of the component (E) relative to 1 part by mass of the component (A) may be 0.6 parts by mass or less, or 0.4 parts by mass or less. If the content of vaseline exceeds 0.8 parts by mass, it becomes difficult to apply the composition thickly. The content of the component (E) relative to 1 part by mass of the component (A) may be 0.1 parts by mass or greater, 0.2 parts by mass or greater, 0.3 parts by mass or greater, or 0.4 parts by mass or greater. Increasing the amount of the component (E) can intensify the matte finish. Reducing the amount of component (E) can make the composition smooth.

The content of the component (E) relative to the mass of the external-use skin preparation composition may be 5 % by mass or greater, 10 % by mass or greater, 12 % by mass or greater, or 15 % by mass or greater. The content of the component (E) relative to the mass of the external-use skin preparation composition may be 30 % by mass or less, 25 % by mass or less, or 20 % by mass or less.

### (C) Liquid-State Oily Component:

In addition to the aforementioned components, the external-use skin preparation composition according to the second embodiment may further include a liquid-state oily component.

In the second embodiment, the content of the component (C) relative to the mass of the external-use skin preparation composition may be 5 % by mass or greater, 10 % by mass or greater, 15 % by mass or greater, 20 % by mass or greater, or 25 % by mass or greater. The content of the component (C) relative to the mass of the external-use skin preparation composition may be 30 % by mass or less, 25 % by mass or less, 20 % by mass or less, 15 % by mass or less, or 10 % by mass or less.

Modes of the component (C) of the second embodiment other than the above can be the same as those in the first embodiment. As regards the modes of the component (C) of the second embodiment other than the above, the description of the first embodiment is incorporated herein by reference, and description thereon is omitted herefrom.

### Common Modes:

Modes common between the first embodiment and the second embodiment will be described below.

### (F) Powder:

In addition to the aforementioned components, the external-use skin preparation composition of the present disclosure may further include a powder.

The powder is not particularly limited, so long as it can be used for general purposes, such as cosmetic uses, etc. Examples of the powder may include inorganic powder (such as talc, kaolin, mica, sericite, muscovite, phlogopite, synthetic mica, lepidolite, biotite, vermiculite, magnesium carbonate, calcium carbonate, aluminum silicate, barium silicate, calcium silicate, magnesium silicate, strontium silicate, tungstate, magnesium, silica, zeolite, glass, barium sulfate, calcined calcium sulfate (calcined gypsum), calcium phosphate, fluorine apatite, hydroxyapatite, ceramic powder, metallic soap (such as zinc myristate, calcium palimitate, and aluminum stearate), and boron nitride, etc); organic powder (such as polyamide resin powder (nylon powder), polyethylene powder, polymethylmethacrylate powder, polystyrene powder, styrene-acrylic acid copolymer powder, benzoguanamine resin powder, poly(tetrafluroethylene) powder, and cellulose powder, silicone resin powder, silk powder, wool powder, urethane powder, etc); inorganic white family pigment (such as titanium dioxide, zinc oxide, etc); inorganic red family pigment (such as iron oxide (colcothar), iron titanate, etc); inorganic brown family pigment (such as γ-iron oxide, etc); inorganic yellow family pigment (such as yellow iron oxide, loess, etc); inorganic black family pigment (such as black iron oxide, carbon black, lower titanium oxide, etc); inorganic purple family pigment (such as manganese violet, cobalt violet, etc); inorganic green family pigment (such as chrome oxide, chrome hydroxide, cobalt titanate, etc); inorganic blue family pigment (such as ultramarine, iron blue, etc); pearl pigment (such as titanium oxide coated mica, titanium oxide coated bismuth oxychloride, titanium oxide coated talc, colored titanium oxide coated mica, bismuth oxychloride, argentine, etc); metal powder pigment (such as aluminum powder, copper powder, etc); organic pigment such as zirconium, barium, or aluminum lake (such as organic pigment such as Red No.201, Red No.202, Red No.204, Red No.205, Red No.220, Red No.226, Red No.228, Red No.405, Red No.201, Orange No.203, Orange No.204, Yellow No.205, Yellow No.401, Blue No.401, Red No.3, Red No. 104, Red No. 106, Red No.227, Red No.230, Red No.401, Red No.505, Orange No.205, Yellow No.4, Yellow No.5, Yellow No.202, Yellow No.203, Green No.3, and Blue No.1, etc); natural pigment (such as chlorophyll, β-carotene, etc); and the like.

The powder can be selected as appropriate depending on the use of the external-use skin preparation. For example, by selecting mica as the powder, the matte finish of the coating region can be improved. By selecting a pigment, the external-use skin preparation composition can be colored.

The content of the component (F) relative to the mass of the external-use skin preparation composition may be 0.1 % by mass or greater, 0.5 % by mass or greater, 1 % by mass or greater, 2 % by mass or greater, 5 % by mass or greater, or 10 % by mass or greater. The content of the component (F) relative to the mass of the external-use skin preparation composition may be 15 % by mass or less, 10 % by mass or less, 5 % by mass or less, or 3 % by mass or less.

### (G) Others:

If necessary, the external-use skin preparation composition of the present disclosure may contain other components as appropriate, such as water, water-soluble alcohols, oily components other than the above, powders, anionic surfactants, cationic surfactants, amphoteric surfactants, hydrophilic nonionic surfactants, lipophilic nonionic surfactants, thickeners, moisturizers, film-forming agents, oil-soluble UV absorbers, water-soluble UV absorbers, metal ion sequestering agents, amino acids, organic amines, polymer emulsions, pH adjusters, skin nutrients, vitamins, antioxidants, antioxidant aids, perfumes, etc., in amounts that do not inhibit the effects of the present disclosure.

Examples of water usable herein may include water used for cosmetics, quasi-pharmaceutical products, or the like, and usable examples may include purified water, ion-exchanged water, tap water, etc.

The content of water relative to the mass of the external-use skin preparation composition is preferably 5 % by mass or less, preferably 3 % by mass or less, more preferably 1 % by mass or less. It is further preferred that the external-use skin preparation composition does not include a substantial amount of water.

Examples of the water-soluble alcohol may include at least one selected from, for example, lower alcohols, polyols, polyol polymers, divalent alcohol alkyl ethers, divalent alcohol alkyl ethers, divalent alcohol ether esters, glycerin monoalkyl ethers, sugar alcohols, monosaccharides, oligosaccharides, polysaccharides, and derivatives thereof.

Examples of the lower alcohol may include ethanol, propanol, isopropanol, and the like.

Examples of the polyhydric alcohol may include dihydric alcohol (such as ethylene glycol, propylen glycol, trimethylene glycol, 1,2-butylene glycol, 1,3-butylene glycol, tetramethylene glycol, 2,3-butylene glycol, pentamethylene glycol, 2-butene-1,4-diol, hexylene glycol, octylene glycol, 1,2-hexanediol, etc); trihydric alcohol (such as glycerin, trimethylolpropane, etc); tetrahydric alcohol (such as such as pentaerythritol such as 1,2,6-hexanetriol, etc); pentahydric alcohol (such as xylitol, etc); hexahydric alcohol (such as sorbitol, mannitol, etc); polyhydric alcohol polymer (such as diethylene glycol, dipropylene glycol, triethylene glycol, polypropylene glycol, tetraethylene glycol, diglycerin, polyethylene glycol, triglycerin, tetraglycerin, polyglycerin, etc); sugar alcohol (such as sorbitol, maltitol, maltotriose, mannitol, sucrose, erythritol, glucose, fructose, starch sugar, maltose, xylitol, starch sugar hydrogenated alcohol, etc); glycolide, tetrahydrofurfuryl alcohol; POE-tetrahydrofurfuryl alcohol; POP/POE-butyl ether; tripolyoxypropylene glycerin ether; POP-glycerin ether; POP-glycerin ether phosphoric acid; POP/POE-pentaerythritol ether; polyglycerin, and the like.

Examples of the oligosaccharide may include at least one selected from sucrose, guntianose, umbelliferose, lactose, planteose, isolignoses, α,α-trehalose, raffinose, lignoses, umbilicin, stachyose, verbascoses, and the like.

Examples of the polysaccharide may include at least one selected from cellulose, quince seed, chondroitinsulfate, starch, galactan, dermatan sulfate, glycogen, acasia gum, heparansulfate, hyaluronan, gum tragacanth, keratan sulfate, chondoroitin, xanthan gum, mucoitin sulfate, guar gum, dextran, keratosulfate, locust bean gum, succinoglycan, caronic acid, and the like.

Examples of other polyols may include at least one polyol selected from polyoxyethylene methyl glucoside (Glucam E-10), polyoxypropylene methyl glucoside (Glucam P-10), and the like.

Examples of usable oily components may include waxes, liquid oils, solid fats, wax esters, higher fatty acids, etc.

Examples of the waxes that may be used may include beeswax, candelilla wax, cotton wax, carnauba wax, bayberry wax, insect wax, montan wax, bran wax, kapok wax, sugarcane wax, hexyl laurate, jojoba wax, shellac wax, POE lanolin alcohol ether, POE cholesterol ether, POE hydrogenated lanolin alcohol ether, liquid paraffin, ozocerite, squalane, pristane, paraffin, ceresin. squalene, microcrystalline wax, Físcher-Trópsch wax, for example, and the like.

Examples of the liquid oil that may be used may include avocado oil, camellia oil, macadamia nut oil, corn oil, olive oil, rapeseed oil, egg yolk oil, sesame oil, par chic oil, wheat germ oil, southern piece oil, castor oil, linseed oil, safflower oil, cotton seed oil, perilla oil, soybean oil, groundnut oil, brown real oil, torreya oil, rice bran oil, Chinese tung oil, Japanese tung oil, jojoba oil, germ oil, triglycerol, and the like.

Examples of the solid fat that may be used may include cacao butter, coconut oil, hydrogenated coconut oil, palm oil, palm kernel oil, Japan wax kernel oil, hardened oil, Japan wax, hydrogenated caster oil, and the like.

Examples of the higher fatty acid that may be used may include lauric acid, myristic acid, palmitic acid, stearic acid, behenic acid, oleic acid, undecylenic acid, tallic acid, isostearic acid, linoleic acid, linolenic acid, eicosapentaenoic acid (EPA), docosahexaenoic acid (DHA) and the like.

Examples of the lipophilic nonionic surfactants may include sorbitan fatty acid ester (such as sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2 ethylhexylate, diglycerol sorbitan tetra-2 ethylhexylate, etc); glyceryl polyglyceryl fatty acid (such as glyceryl monocotton oil fatty acid, glyceryl monoerucate, glyceryl sesquioleate, glyceryl monostearate, glyceryl α, α'-oleate pyroglutamate, glyceryl monostearate malate, etc); propylene glycol fatty acid ester (such as propylene glycol monostearate, etc); hydrogenated caster oil derivative; glyceryl alkyl ether, and the like.

Examples of the natural water-soluble polymer may include plant-based polymer (such as gum Arabic, gum tragacanth, galactan, guar gum, locust bean gum, gum karaya, carrageenan, pectine, agar, quince seed (cydonia oblonga), algae colloid (brown algae extract), starch (rice, corn, potato, wheat) ,glicyrrhizic acid); microorganism based polymer (such as xanthan gum, dextran, succinoglycan, pullulan, etc), and the like.

Examples of the semisynthetic water-soluble polymer may include starch-based polymer (such as carboxymethyl starch, methylhydroxypropyl starch, etc); cellulose-based polymer (such as methylcellulose, ethylcellulose, methylhydroxypropylcellulose, hydroxyethylcellulose, cellulose sodium sulfate, hydroxypropylcellulose, carboxymethylcellulose, sodium calboxymethyl cellulose, crystalline cellulose, cellulose powder, etc); algin acid-based polymer (such as sodium alginate, propylene glycol alginate ester, etc), and the like.

Examples of the thickeners may include gum arabic, carrageenan, karaya gum, tragacanth gum, carob gum, quince seed (marmelo), casein, dextrin, gelatin, sodium pectate, sodium alginate, methyl cellulose, ethyl cellulose, carboxymethyl cellulose (CMC), hydroxyethyl cellulose, hydroxypropyl cellulose, polyvinyl alcohol (PVA), polyvinylmethyl ether (PVM), PVP (polyvinyl pyrrolidone), polysodium acrylate, carboxyvinyl polymer, locust bean gum, guar gum, tamarind gum, dialkyldimethylammonium sulfate cellulose, xanthan gum, aluminum magnesium silicate, bentonite, hectorite, aluminum magnesium silicate (Veegum), sodium magnesium silicate (Laponite), silicic acid anhydride, taurate-based synthetic polymers, and acrylate-based synthetic polymers, and the like.

Examples of the moisturizers may include polyethylene glycol, propylene glycol, glycerin, 1,3-butylene glycol, xylitol, sorbitol, maltitol, diglycerin (EO)PO adduct, chestnut rose extract, yarrow extract, melilot extract, and the like.

Examples of the film-forming agent may include polymeric silicone, silicone resin, trimethylsiloxysilicate, and the like.

Examples of the vitamins may include vitamine A, B 1, B2, B6, C, E and derivatives thereof, pantothenic acid and derivatives thereof, biotin, and the like.

Examples of the anti-oxidant may include tocopherols, dibutyl hydroxy toluene, butyl hydroxy anisole, and gallic acid esters, and the like.

Examples of the anti-oxidant aid may include phosphoric acid, citric acid, ascorbic acid, maleic acid, malonic acid, succinic acid, fumaric acid, cephalin, hexamethaphosphate, phytic acid, ethylenediaminetetraacetic acid, and the like.

Examples of other containable compositions may include an antiseptic agent (such as ethylparaben, butylparaben, chlorphenesin, 2-phenoxyethanol, etc); antiphlogistic (such as glycyrrhizinic acid derivatives, glycyrrhetic acid derivatives, salicylic acid derivatives, hinokitiol, zinc oxide, allantoin, etc); a skin-whitening agent (such as placental extract, saxifrage extract, arbutin, etc); various extracts (such as phellodendron bark (cork tree bark), coptis rhizome, lithospermum, peony, swertia herb, birch, sage, loquat, carrot, aloe, mallow, iris, grape, coix seed, sponge gourd, lily, saffron, cnidium rhizome, ginger, hypericum, restharrow, garlic, red pepper, citrus unshiu, Japanese angelica, seaweed, etc); an activator (such as royal jelly, photosenstizer, cholesterol derivatives, etc); a blood circulation promotion agent (such as nonylic acid vanillylamide, nicotine acid benzyl ester, nicotine acid β-butoxyethyl ester, capsaicin, zingerone, cantharides tincture, ichthammol, tannic acid, α-borneol, tocopheryl nicotinate, meso-inositol hexanicotinate, cyclandelate, cinnarizine, tolazoline, acetylcholine, verapamil, cepharanthine, γ-oryzanol, etc); an antiseborrheric agent, (such as sulfur, thianthl, etc); an anti-inflammatory agent (such as tranexamic acid, thiotaurine, hypotaurine, etc), and the like.

The composition of the present disclosure may further contain, as appropriate: caffeine, tannin, verapamil, tranexamic acid and derivatives thereof; various herbal medicine extracts such as licorice, pseudocydonia sinensis, pyrola japonica, etc.; drugs such as tocopherol acetate, glycyrrhetinic acid, glycyrrhizinic acid and derivatives or salts thereof; skin whitening agents such as vitamin C, magnesium ascorbyl phosphate, ascorbic acid glucoside, arbutin, kojic acid, etc.; amino acids such as arginine, lysine, etc., and derivatives thereof.

### Form and Hardness:

The external-use skin preparation composition of the present disclosure may have a cream form or a paste form. A cream-form or paste-form external-use skin preparation composition may be contained in a tube-type container.

It is preferred that the external-use skin preparation composition of the present disclosure has a hardness of 8 or greater. It is preferred that the external-use skin preparation composition of the present disclosure has a hardness of 200 or less. In cases where the external-use skin preparation composition is made in a cream form or paste form, it is preferred that the external-use skin preparation composition has a hardness of 30 or greater. It is also preferred that the external-use skin preparation composition has a hardness of 100 or less.

Hardness can be measured by filling a measurement container with the external-use skin preparation composition immediately after manufacture, leaving it to stand for 1 hour, and then using Rheo Meter from Sun Scientific Co., Ltd. to measure the hardness under the following conditions: load: 2 kg; needle diameter: 5.6 ϕ; penetration depth: 3 mm; rising speed: 20 mm/min.; measurement temperature: 25°C.

### Manufacturing Method:

A method for manufacturing the external-use skin preparation composition of the present disclosure will be described. The external-use skin preparation composition of the present disclosure can be manufacturing according to a known method. For example, microcrystalline wax is heated and molten. Next, the other components are added to and mixed with the molten microcrystalline wax. Next, the mixture is cooled. In this way, the external-use skin preparation composition of the present disclosure can be manufactured. By first mixing the heated and molten microcrystalline wax and liquid-state oily component(s) and then solidifying the mixture, it is possible to manufacture a uniform composition having a moderate hardness.

There may be cases where it is difficult, or utterly impractical, to directly define the external-use skin preparation composition of the present disclosure based on the compositional makeup, structure, etc., thereof. In such circumstances, it should be permissible to define the external-use skin preparation composition of the present disclosure according to methods for producing the same.

The external-use skin preparation composition of the present disclosure is applicable, for example, to moisturizers, cosmetics, etc. For example, the external-use skin preparation composition of the present disclosure is applicable to lip moisturizers (lip creams), skin moisturizers (balms), lip rouge, lip gloss, etc.

The external-use skin preparation composition of the present disclosure can achieve a lusterless matte finish in a region where the composition is applied. Further, the external-use skin preparation composition of the present disclosure can be applied thickly to the skin, even without exhibiting a matte finish. Furthermore, the external-use skin preparation composition of the present disclosure is smooth and can thus be applied with a uniform thickness. Thus, in cases where, for example, the external-use skin preparation composition of the present disclosure is used as a moisturizer, the composition can be applied thickly to the skin with a uniform thickness, thereby achieving excellent, lasting moisturizing effects. Further, the external-use skin preparation composition of the present disclosure has suppressed stickiness, even though it can be applied thickly.

### Examples

The external-use skin preparation composition of the present disclosure will be described below by way of examples. The external-use skin preparation composition of the present disclosure is, however, not limited to the following examples. The content by percentage of each of the components shown in the Tables is in terms of percent by mass (% by mass).

### Test Examples 1 to 9:

External-use skin preparation compositions with different types of solid-form oily gelling agents were prepared. For each external-use skin preparation composition, the bulk hardness was measured, and the following evaluations were performed: whether the coating film had a matte finish; whether the coating film was thick; whether the coating feel was smooth; whether the external-use skin preparation composition could be applied evenly; in which type of container, i.e., a tube container or a bottle container, the external-use skin preparation composition could be suitably contained; and stability in a high-temperature environment. The evaluation methods and evaluation criteria for these test items are described below. Tables 1 and 2 show the compositional makeup and evaluation for each of the external-use skin preparation compositions.

### Bulk Hardness:

A measurement container was filled with the external-use skin preparation composition immediately after manufacture, and it was left to stand for 1 hour. Then, the hardness of the external-use skin preparation composition was measured using Rheo Meter from Sun Scientific Co., Ltd. under the following conditions: load: 2 kg; needle diameter: 5.6 ϕ; penetration depth: 3 mm; rising speed: 20 mm/min.; measurement temperature: 25°C.

### Evaluation of Feel upon Use and Finish:

Ten expert panelists applied each sample back and forth 1.5 times to the lips, to evaluate the feel upon use and the finish (presence/absence of matte finish; thickness of coating film; smoothness; lack of unevenness; and lack of stickiness). Each evaluation item was rated as follows according to the number of panelists.
A: Nine or more panelists rated the sample as "good" in relation to the evaluation item.
B: Seven to eight panelists rated the sample as "good" in relation to the evaluation item.
C: Six panelists rated the sample as "good" in relation to the evaluation item.
D: Four to five panelists rated the sample as "good" in relation to the evaluation item.
E: Three or fewer panelists rated the sample as "good" in relation to the evaluation item.

### Type of Container:

T: The external-use skin preparation composition is usable in a tube-type container.
B: The external-use skin preparation composition is usable in a bottle-type and/or jar-type container.

### High-Temperature Stability:

Each external-use skin preparation composition was left to stand in a 50°C environment for 2 weeks, and the appearance of the external-use skin preparation composition was evaluated according to the following criterion.
Good: Neither separation nor melting of the external-use skin composition was observed.
Poor: The external-use skin composition separated or melted.

The external-use skin preparation composition of Test Example 1 which did not include a solid-form oily gelling agent (component (B)) had a high rating in terms of matte finish, but had low ratings in terms of coating film thickness, lack of unevenness and lack of stickiness. In contrast, the external-use skin preparation compositions of Test Examples 2 to 8 which included solid-form oily gelling agents had high ratings regarding all of the evaluation items. Test Example 9 which included dextrin palmitate as the solid-form oily gelling agent, however, had low ratings in terms of matte finish and coating film thickness. From the above, it is thought that at least the solid-form oily gelling agents used in Test Examples 2 to 8 can prepare favorable external-use skin preparation compositions.

The external-use skin preparation compositions of Test Examples 2 to 8 were easy to take out from a tube-type container, and could also be taken out from a bottle-type container with no problem.

**[Table 1]**

| Test Example | | 1 | 2 | 3 | 4 | 5 |
|---|---|---|---|---|---|---|
| (A) Microcrystalline wax | | 33 | 33 | 33 | 33 | 33 |
| (B) Glyceryl behenate/eicosadioate | | - | 1.2 | - | - | - |
| (B) Hydrogenated vegetable oil | | - | - | 1.2 | - | - |
| (B) Jojoba ester | | - | - | - | 1.2 | - |
| (B) Behenyl alcohol | | - | - | - | - | 1.2 |
| (B) Stearyl alcohol | | - | - | - | - | - |
| (B) Castor oil/IPID copolymer | | - | - | - | - | - |
| Caprylic/capric triglyceride | | - | - | - | - | - |
| (B) Glyceryl behenate | | - | - | - | - | - |
| Polyglyceryl-6 octastearate | | - | - | - | - | - |
| (B') Dextrin palmitate | | - | - | - | - | - |
| (C) Pentaerythrityl tetraethylhexanoate | | 27 | 25.8 | 25.8 | 25.8 | 25.8 |
| (D) Hydrogenated polydecene | | 40 | 40 | 40 | 40 | 40 |
| Total | | 100 | 100 | 100 | 100 | 100 |
| Hardness | Bulk hardness | 27 | 38 | 32 | 31 | 27 |
| Evaluation | Matte finish | A | A | A | A | A |
| | Thickness of coating film | D | A | A | A | A |
| | Smoothness | A | A | A | A | A |
| | Lack of unevenness | E | A | A | A | A |
| | Lack of stickiness | E | A | B | A | A |
| | Container type | T&B | T&B | T&B | T&B | T&B |

**[Table 2]**

| Test Example | | 6 | 7 | 8 | 9 |
|---|---|---|---|---|---|
| (A) Microcrystalline wax | | 33 | 33 | 33 | 33 |
| (B) Glyceryl behenate/eicosadioate | | - | - | - | - |
| (B) Hydrogenated vegetable oil | | - | - | - | - |
| (B) Jojoba ester | | - | - | - | - |
| (B) Behenyl alcohol | | - | - | - | - |
| (B) Stearyl alcohol | | 1.2 | - | - | - |
| (B) Castor oil/IPID copolymer | | - | 1.2 | - | - |
| Caprylic/capric triglyceride | | | | | |
| (B) Glyceryl behenate | | - | - | 1.2 | - |
| Polyglyceryl-6 octastearate | | | | | |
| (B') Dextrin palmitate | | - | - | - | 1.2 |
| (C) Pentaerythrityl tetraethylhexanoate | | 27 | 25.8 | 25.8 | 25.8 |
| (D) Hydrogenated polydecene | | 40 | 40 | 40 | 40 |
| Total | | 100 | 100 | 100 | 100 |
| Hardness | Bulk hardness | 25 | 29 | 32 | 13 |
| Evaluation | Matte finish | A | A | A | E |
| | Thickness of coating film | C | C | A | E |
| | Smoothness | A | A | A | A |
| | Lack of unevenness | A | A | A | A |
| | Lack of stickiness | A | A | A | A |
| | Container type | T&B | T&B | T&B | T&B |

### Test Examples 10 to 18:

External-use skin preparation compositions with different types and amounts of waxes were prepared. For each external-use skin preparation composition, the same tests as those for Test Examples 1 to 9 were conducted. Tables 3 and 4 show the compositional makeup and evaluation for each of the external-use skin preparation compositions.

All of the external-use skin preparation compositions of Test Examples 10 to 18 had low ratings in terms of matte finish. Also, the external-use skin preparation compositions of Test Examples 10 to 15, 17 and 18 could not make the coating film thick. Furthermore, some Test Examples had low ratings in terms of lack of unevenness. From the above, it is thought that microcrystalline wax is a preferable wax. It is also thought that the content by percentage of microcrystalline wax is preferably 10 % by mass or greater.

**[Table 3]**

| Test Example | | 10 | 11 | 12 | 13 | 14 |
|---|---|---|---|---|---|---|
| (A) Microcrystalline wax | | 0.75 | 1.0 | 1.5 | 0.5 | - |
| (A') Polyethylene wax | | 4.25 | 4.0 | 6.0 | - | - |
| (A') Paraffin wax | | - | - | - | 4.5 | - |
| (A') Candelilla wax | | - | - | - | - | 10 |
| (A') Beeswax | | - | - | - | - | - |
| (A') Synthetic wax | | - | - | - | - | - |
| (A') Carnauba wax | | - | - | - | - | - |
| (B) Glyceryl behenate/eicosadioate | | 1.2 | 1.2 | 1.2 | 1.2 | 1.2 |
| (C) Pentaerythrityl tetraethylhexanoate | | 53.8 | 53.8 | 51.3 | 53.8 | 48.8 |
| (D) Hydrogenated polydecene | | 40 | 40 | 40 | 40 | 40 |
| Total | | 100 | 100 | 100 | 100 | 100 |
| Hardness | Bulk hardness | 20 | 7 | 103 | 25 | 181 |
| Evaluation | Matte finish | E | E | E | E | E |
| | Thickness of coating film | E | E | E | E | E |
| | Smoothness | A | A | A | A | A |
| | Lack of unevenness | A | A | A | A | A |
| | Lack of stickiness | A | A | A | A | A |
| | Container type | T&B | B | B | T&B | B |

**[Table 4]**

| Test Example | | 15 | 16 | 17 | 18 |
|---|---|---|---|---|---|
| (A) Microcrystalline wax | | - | - | - | - |
| (A') Polyethylene wax | | - | - | - | - |
| (A') Paraffin wax | | - | - | - | - |
| (A') Candelilla wax | | - | - | - | - |
| (A') Beeswax | | 10 | 30 | - | - |
| (A') Synthetic wax | | - | - | 5 | - |
| (A') Carnauba wax | | - | - | - | 10 |
| (B) Glyceryl behenate/eicosadioate | | 1.2 | 1.2 | 1.2 | 1.2 |
| (C) Pentaerythrityl tetraethylhexanoate | | 48.8 | 28.8 | 53.8 | 48.8 |
| (D) Hydrogenated polydecene | | 40 | 40 | 40 | 40 |
| Total | | 100 | 100 | 100 | 100 |
| Hardness | Bulk hardness | Liquid-state | 37 | 19 | 30 |
| Evaluation | Matte finish | E | E | E | E |
| | Thickness of coating film | E | C | E | E |
| | Smoothness | A | E | A | E |
| | Lack of unevenness | A | E | A | E |
| | Lack of stickiness | A | A | A | A |
| | Container type | B | T&B | T&B | T&B |

### Test Examples 19 to 24:

External-use skin preparation compositions with different mass ratios between microcrystalline wax and solid-form oily gelling agents were prepared. For each external-use skin preparation composition, the same tests as those for Test Examples 1 to 9 were conducted. Table 5 shows the compositional makeup and evaluation for each of the external-use skin preparation compositions.

In all of Test Examples 19 to 24, it was possible to achieve favorable matte finish and film thickness. From the above, it is thought that the content of the solid-form oily gelling agent relative to 1 part by mass of microcrystalline wax can be 0.0015 parts by mass or greater. A tendency was observed wherein the smoothness improved with an increase in the proportion of the solid-form oily gelling agent. It is thought that the content of the solid-form oily gelling agent relative to 1 part by mass of microcrystalline wax may be at least 0.7 parts by mass or less. A tendency was observed wherein ratings in terms of matte finish and coating film thickness improved with a decrease in the proportion of the solid-form oily gelling agent.

**[Table 5]**

| Test Example | | 19 | 20 | 21 | 22 | 23 | 24 |
|---|---|---|---|---|---|---|---|
| (A) Microcrystalline wax | | 40 | 40 | 35 | 30 | 25 | 20 |
| (B) Glyceryl behenate/eicosadioate | | 0.1 | 0.2 | 0.5 | 1.4 | - | - |
| (B) Stearyl alcohol | | - | - | - | - | 5 | 10 |
| (C) Pentaerythrityl tetraethylhexanoate | | 19.9 | 19.8 | 24.5 | 28.6 | 30 | 30 |
| (D) Hydrogenated polydecene | | 40 | 40 | 40 | 40 | 40 | 40 |
| Total | | 100 | 100 | 100 | 100 | 100 | 100 |
| (B)/(A) | | 0.0025 | 0.005 | 0.014 | 0.047 | 0.2 | 0.5 |
| Hardness | Bulk hardness | 70 | 56 | 46 | 33 | 40 | 92 |
| Evaluation | Matte finish | A | A | A | A | B | C |
| | Thickness of coating film | A | A | A | B | B | B |
| | Smoothness | B | B | B | A | A | A |
| | Lack of unevenness | A | A | A | A | A | A |
| | Lack of stickiness | A | A | A | A | A | A |
| | Container type | B | B | T&B | T&B | T&B | B |

### Test Examples 25 to 34:

External-use skin preparation compositions were prepared using liquid-state oily components (C) different from the liquid-state oily components used in the aforementioned Test Examples. For each external-use skin preparation composition, the same tests as those for Test Examples 1 to 9 were conducted. Tables 6 and 7 show the compositional makeup and evaluation for each of the external-use skin preparation compositions.

Test Example 25 which did not included any liquid-state ester oil, liquid-state silicone oil, nor liquid-state higher alcohol had low hardness and a low rating in terms of coating film thickness. In contrast, Test Examples 26 to 34 which included a liquid-state ester oil, a liquid-state silicone oil, or a liquid-state higher alcohol were able to obtain good ratings regarding all of the evaluation items. From the above, it is thought that the liquid-state oily component, other than liquid-state hydrocarbon oil, is preferably a liquid-state oily component capable of increasing the hardness of the composition by being mixed with microcrystalline wax (see Test Examples below). It is thought that, for such a liquid-state oily component, it is preferred to use at least one of liquid-state ester oils, liquid-state silicone oils and liquid-state higher alcohols.

It was found that the content of the liquid-state oily component, which is the component (C), can be 2 % by mass or greater relative to the mass of the composition. It is thought that the content of the liquid-state oily component (C) relative to the mass of the composition is preferably 75 % by mass or less, more preferably 65 % by mass or less.

Test Examples 31 and 34 were able to obtain good ratings even without including a liquid-state hydrocarbon oil (D). From the above, it was found that, in embodiments including a liquid-state oily component (C), it is not absolutely necessary to include a liquid-state hydrocarbon oil (D). A tendency was observed wherein the rating regarding smoothness improved when a liquid-state hydrocarbon oil (D) was included. It is thought that the content of the liquid-state hydrocarbon oil (D) relative to the mass of the composition is preferably 5 % by mass or greater. The content of the liquid-state hydrocarbon oil (D) relative to the mass of the composition can be 70 % by mass or less.

**[Table 6]**

| Test Example | | 25 | 26 | 27 | 28 | 29 |
|---|---|---|---|---|---|---|
| (A) Microcrystalline wax | | 33 | 33 | 33 | 33 | 33 |
| (B) Glyceryl behenate/eicosadioate | | 1.2 | 1.2 | 1.2 | 1.2 | 1.2 |
| (C) Diphenylsiloxy phenyl trimethicone | | - | 25.8 | - | - | - |
| (C) Polysilicone-15 | | - | - | 5.8 | 15.8- | - |
| (C) Neopentyl glycol dicaprate | | - | - | - | - | 45.8 |
| (C) Octyldodecanol | | - | - | - | - | - |
| (D) Hydrogenated polydecene | | 65.8 | 40 | 60 | 50 | 20 |
| Total | | 100 | 100 | 100 | 100 | 100 |
| (D)/(C) | | - | 1.55 | 10.34 | 3.16 | 0.44 |
| Hardness | Bulk hardness | 5 | 44 | 69 | 126 | 54 |
| Evaluation | Matte finish | B | A | A | A | A |
| | Thickness of coating film | E | A | A | A | A |
| | Smoothness | A | A | A | A | A |
| | Lack of unevenness | A | A | A | A | A |
| | Lack of stickiness | A | A | A | A | A |
| | Container type | T&B | T&B | T&B | B | T&B |

**[Table 7]**

| Test Example | | 30 | 31 | 32 | 33 | 34 |
|---|---|---|---|---|---|---|
| (A) Microcrystalline wax | | 33 | 33 | 33 | 33 | 33 |
| (B) Glyceryl behenate/eicosadioate | | 1.2 | 1.2 | 1.2 | 1.2 | 1.2 |
| (C) Diphenylsiloxy phenyl trimethicone | | - | - | - | - | - |
| (C) Polysilicone-15 | | - | - | - | - | - |
| (C) Neopentyl glycol dicaprate | | 55.8 | 65.8 | - | - | - |
| (C) Octyldodecanol | | - | - | 45.8 | 55.8 | 65.8 |
| (D) Hydrogenated polydecene | | 10 | - | 20 | 10 | - |
| Total | | 100 | 100 | 100 | 100 | 100 |
| (D)/(C) | | 0.18 | 0 | 0.44 | 0.18 | 0 |
| Hardness | Bulk hardness | 80 | 155 | 57 | 75 | 116 |
| Evaluation | Matte finish | A | B | A | A | A |
| | Thickness of coating film | A | A | A | A | A |
| | Smoothness | A | B | A | A | B |
| | Lack of unevenness | A | A | A | A | A |
| | Lack of stickiness | A | A | A | A | A |
| | Container type | T&B | B | T&B | T&B | B |

### Test Examples 35 to 40:

External-use skin preparation compositions including vaseline were prepared. For each external-use skin preparation composition, the same tests as those for Test Examples 1 to 9 were conducted. Table 8 shows the compositional makeup and evaluation for each of the external-use skin preparation compositions.

The external-use skin preparation composition of Test Example 35 which did not contain microcrystalline wax nor solid-form oily gelling agent had low ratings in terms of matte finish and coating film thickness. High-temperature stability was also poor. In Test Example 36, the ratings did not change even though a solid-form oily gelling agent was added to the composition of Test Example 35. From the above, it was found that vaseline cannot serve as a substitute for microcrystalline wax. In Test Examples 37 and 38, a portion of vaseline in Test Example 36 was replaced by microcrystalline wax. As a result, the coating film thickness and high-temperature stability improved, but in Test Example 37, the rating regarding coating film thickness was still low. From the above, it is thought that, in embodiments including vaseline, the content of microcrystalline wax is preferably 20 % by mass or greater. It is thought that the content of vaseline relative to 1 part by mass of microcrystalline wax is preferably 0.8 parts by mass or less.

Test Examples 39 and 40, in which a portion of the liquid-state oily component (C) in Test Example 36 was replaced by vaseline, had good ratings. Particularly in Test Example 40, there was no problem even though no liquid-state oily component (C) was included. From the above, it is thought that the content of vaseline can be 30 % by mass or less.

**[Table 8]**

| Test Example | | 35 | 36 | 37 | 38 | 39 | 40 |
|---|---|---|---|---|---|---|---|
| (A) Microcrystalline wax | | - | - | 16 | 25 | 33 | 33 |
| (B) Glyceryl behenate/eicosadioate | | - | 1.2 | 1.2 | 1.2 | 1.2 | 1.2 |
| (C) Pentaerythrityl tetraethylhexanoate | | 25.8 | 25.8 | 25.8 | 25.8 | 10.8 | - |
| (D) Hydrogenated polydecene | | 40 | 40 | 40 | 40 | 40 | 40 |
| (E) Vaseline | | 34.2 | 33 | 17 | 8 | 15 | 25.8 |
| Total | | 100 | 100 | 100 | 100 | 100 | 100 |
| (E)/(A) | | - | - | 1.06 | 0.32 | 0.45 | 0.78 |
| Hardness | Bulk hardness | 2 | 4 | 8 | 36 | 125 | 99 |
| Evaluation | Matte finish | E | E | A | A | A | A |
| | Thickness of coating film | E | E | D | A | A | A |
| | Smoothness | A | A | A | A | C | C |
| | Lack of unevenness | A | A | A | A | A | A |
| | Lack of stickiness | A | A | A | A | A | A |
| | Container type | B | B | B | T&B | B | T&B |
| | High-temperature stability | Poor | Poor | Good | Good | Good | Good |

### Test Examples 41 to 44:

External-use skin preparation compositions including a powder were prepared. For each external-use skin preparation composition, the same tests as those for Test Examples 1 to 9 were conducted. Table 9 shows the compositional makeup and evaluation for each of the external-use skin preparation compositions.

The external-use skin preparation compositions including a powder were also able to obtain good ratings. It is thought that the powder can be added at least up to 15 % by mass. In Test Example 42, it was possible to further improve matte finish. From the above, it is thought that, in cases where matte finish is to be improved, it is preferred to add at least 5 % by mass of mica. Further, in Test Examples 43 and 44, there was no problem even though a pigment was added. This shows that the external-use skin preparation composition of the present disclosure is applicable to cosmetics such as lip rouge, etc.

**[Table 9]**

| Test Example | | 41 | 42 | 43 | 44 |
|---|---|---|---|---|---|
| (A) Microcrystalline wax | | 33 | 33 | 33 | 33 |
| (B) Glyceryl behenate/eicosadioate | | 1.2 | 1.2 | 1.2 | 1.2 |
| (C) Pentaerythrityl tetraethylhexanoate | | 25.3 | 15.8 | 20.8 | - |
| (C) Diisostearyl malate | | 0.2 | - | 2 | 10.3 |
| (C) Glyceryl diisostearate | | 0.1 | - | 1 | 5.2 |
| (D) Hydrogenated polydecene | | 40 | 40 | 40 | 40 |
| (F) Mica | | 0.2 | 10 | - | - |
| (F) Red pigment | | - | - | 2 | 10.3 |
| Total | | 100 | 100 | 100 | 100 |
| Hardness | Bulk hardness | 59 | 82 | 69 | 150 |
| Evaluation | Matte finish | A | A | A | A |
| | Thickness of coating film | A | A | A | A |
| | Smoothness | A | A | A | B |
| | Lack of unevenness | A | A | A | A |
| | Lack of stickiness | A | A | A | A |
| | Container type | T&B | T&B | T&B | B |

### Test Examples 45:

Liquid-state oily components that, by being mixed with the microcrystalline wax (A), can provide a mixture having a moderate hardness were tested. The microcrystalline wax and each of the liquid-state oily components shown in Table 10 were mixed at a mass ratio of 1:3. The mixture was heated until molten, and then mixed, and then the mixture was cooled to room temperature and solidified. The hardness of the mixture was measured using Rheo Meter from Sun Scientific Co., Ltd. under the following conditions: load: 2 kg; penetration depth: 3 mm; rising speed: 20 mm/min.; measurement temperature: 25°C. Needles with diameters of 1 ϕ and 5.6 ϕ were used.

Liquid-state ester oils, liquid-state higher alcohols, and liquid-state silicone oils (except for nonvolatile dimethicone) were capable of producing a mixture, together with microcrystalline wax, having a sufficient hardness and a uniform composition. In this measurement, a hardness over 200 could not be measured. However, it was found that, when the hardness measured with a needle diameter of 1 ϕ was 3 or greater, sufficient hardness could be obtained even with a needle diameter of 5.6 ϕ.

**[Table 10]**

| | Liquid-state oily component | Hardness | |
|---|---|---|---|
| | | 1ϕ | 5.6ϕ |
| Test Example 45-1 | Dimethicone (nonvolatile) | Nonuniform | Nonuniform |
| Test Example 45-2 | Dipropylene glycol | Nonuniform | Nonuniform |
| Test Example 45-3 | Glycerin | Nonuniform | Nonuniform |
| Test Example 45-4 | Squalane | 0 | 5 |
| Test Example 45-5 | Hydrogenated polydecene | 0 | 7 |
| Test Example 45-6 | Mineral oil (low viscosity: 21-25 cSt) | 0 | 9 |
| Test Example 45-7 | Mineral oil (high viscosity: 66-69 cSt) | 0 | 18 |
| Test Example 45-8 | Octyldodecanol | 0 | 36 |
| Test Example 45-9 | Neopentyl glycol dicaprate | 1 | 51 |
| Test Example 45-10 | Isostearic acid | 3 | 124 |
| Test Example 45-11 | Glyceryl diisostearate | 3 | 125 |
| Test Example 45-12 | Diphenylsiloxy phenyl trimethicone | 3 | 189 |
| Test Example 45-13 | Dimethicone (1.5 cs) (volatile) | 5 | 134 |
| Test Example 45-14 | Triisostearin | 9 | - |
| Test Example 45-15 | Triethylhexanoin | 9 | 199 |
| Test Example 45-16 | Diisostearyl malate | 11 | - |
| Test Example 45-17 | Caprylic/capric triglyceride | 13 | 195 |
| Test Example 45-18 | Vaseline | 13 | - |
| Test Example 45-19 | Pentaerythrityl tetraethylhexanoate | 14 | - |
| Test Example 45-20 | Phytosteryl macadamiate | 19 | - |
| Test Example 45-21 | Olea europaea (olive) fruit oil | 19 | - |
| Test Example 45-22 | Phytosteryl isostearyl dimer dilinoleate | 20 | - |
| Test Example 45-23 | Macadamia ternifolia seed oil | 21 | - |
| Test Example 45-24 | Phytosteryl/octyldodecyl lauroyl glutamate | 22 | - |
| Test Example 45-25 | Polyglyceryl-2 triisostearate | 26 | - |
| Test Example 45-26 | Ethylhexyl methoxycinnamate | 30 | - |
| Test Example 45-27 | Polyglyceryl-2 diisostearate | 32 | - |
| Test Example 45-28 | Pentaerythrityl tetrabehenate/benzoate/ethylhexanoate | 35 | - |
| Test Example 45-29 | Sorbitan sesquiisostearate | 44 | - |
| Test Example 45-30 | Macadamia seed oil polyglyceryl-6 esters behenate | 45 | - |
| Test Example 45-31 | Behenyl/phytosteryl dimer dilinoleate | 45 | - |
| Test Example 45-32 | Trimethyl pentaphenyl trisiloxane | 65 | - |
| Test Example 45-33 | Diphenyl dimethicone | 134 | - |
| Test Example 45-34 | Dipentaerythrityl hexahydroxystearate | 144 | - |
| Test Example 45-35 | Polysilicone-15 | 161 | - |

The external-use skin preparation composition and the method for suppressing the precipitation of adenosine according to the present invention have been described according to the foregoing embodiments and examples, but the invention is not limited to the foregoing embodiments and examples and may encompass various transformations, modifications, and improvements made to the various disclosed elements (including elements disclosed in the Claims, Description, and Drawings) within the scope of the invention and according to the fundamental technical idea of the present invention. Further, various combinations, substitutions, and selections of the various disclosed elements are possible within the scope of the claims of the invention.

Further issues, objectives, and embodiments (including modifications) of the present invention are revealed also from the entire disclosure of the invention including the Claims.

The numerical ranges disclosed herein are to be construed in such a manner that arbitrary numerical values and ranges falling within the disclosed ranges are treated as being concretely described herein, even where not specifically stated.

Some or all of the foregoing embodiments may be described as in the following additional features, although not limited thereto. The various additional features may be employed in combination with the claim(s) in the Scope of Claims.

### [Additional Feature 1]

A method of using an external-use skin preparation composition, comprising using the external-use skin preparation composition of the present disclosure as a moisturizer.

### [Additional Feature 2]

A method of using an external-use skin preparation composition, comprising applying the external-use skin preparation composition of the present disclosure to the lips.

### Industrial Applicability

The external-use skin preparation composition of the present disclosure is applicable, for example, to cosmetics, cleansers, etc., applicable to the skin. The oily solid-form cosmetic of the present disclosure is applicable, for example, to base makeup, veil makeup, makeup cosmetics, antiperspirants, deodorants, sun-block cosmetics, skin-care agents, cleansers, etc.

## Claims

1. An external-use skin preparation composition comprising:
(A) from 15 % by mass to 40 % by mass of microcrystalline wax;
(B) a solid-form oily gelling agent including at least one selected from the group consisting of glyceryl behenate/eicosadioate, hydrogenated vegetable oil, jojoba ester, behenyl alcohol, stearyl alcohol, castor oil/IPID copolymer, caprylic/capric triglyceride, glyceryl behenate, and polyglyceryl-6 octastearate; and
(C) from 2 % by mass to 75 % by mass of a liquid-state oily component, the component (C) being a liquid-state oily component wherein a mixture obtained by melt-mixing the component (A) and the component (C) at a mass ratio of 1:3 and then solidifying the same has a 3-mm penetration hardness (needle diameter: 5.6 ϕ) of 30 or greater at 25°C.

2. The external-use skin preparation composition according to claim 1, wherein the component (C) includes at least one selected from the group consisting of liquid-state ester oils, liquid-state higher alcohols, and liquid-state silicone oils.

3. The external-use skin preparation composition according to claim 1 or 2, further comprising
(D) from 5 % by mass to 70 % by mass of a liquid-state hydrocarbon oil.

4. The external-use skin preparation composition according to any one of claims 1 to 3, wherein a content of (E) vaseline is 3 % by mass or less relative to the mass of the external-use skin preparation composition.

5. An external-use skin preparation composition comprising:
(A) from 20 % by mass to 40 % by mass of microcrystalline wax;
(B) a solid-form oily gelling agent including at least one selected from the group consisting of glyceryl behenate/eicosadioate, hydrogenated vegetable oil, jojoba ester, behenyl alcohol, stearyl alcohol, castor oil/IPID copolymer, caprylic/capric triglyceride, glyceryl behenate, and polyglyceryl-6 octastearate;
(D) from 30 % by mass to 50 % by mass of a liquid-state hydrocarbon oil; and
(E) vaseline, wherein a content of the component (E) is 0.8 parts by mass or less relative to 1 part by mass of the component (A).

6. The external-use skin preparation composition according to claim 5, wherein the content of the component (E) is from 5 % by mass to 30 % by mass relative to the mass of the external-use skin preparation composition.

7. The external-use skin preparation composition according to claim 5 or 6, further comprising
(C) from 5 % by mass to 30 % by mass of a liquid-state oily component, the component (C) being a liquid-state oily component wherein a mixture obtained by melt-mixing the component (A) and the component (C) at a mass ratio of 1:3 and then solidifying the same has a 3-mm penetration hardness (needle diameter: 5.6 ϕ) of 30 or greater at 25°C.

8. The external-use skin preparation composition according to any one of claims 5 to 7, wherein the component (C) includes at least one selected from the group consisting of liquid-state ester oils, liquid-state higher alcohols, and liquid-state silicone oils.

9. The external-use skin preparation composition according to any one of claims 1 to 8, wherein a content of the component (B) is from 0.0015 to 0.7 parts by mass relative to 1 part by mass of the component (A).

10. The external-use skin preparation composition according to any one of claims 1 to 9, further comprising
(F) from 0.1 % by mass to 15 % by mass of a powder.

11. The external-use skin preparation composition according to any one of claims 1 to 10, wherein a content of water is 5 % by mass or less relative to the mass of the external-use skin preparation composition.

12. The external-use skin preparation composition according to any one of claims 1 to 11, wherein a hardness measured under the following conditions is from 30 to 100: load: 2 kg; needle diameter: 5.6 ϕ; penetration depth: 3 mm; rising speed: 20 mm/min.; measurement temperature: 25°C.

13. The external-use skin preparation composition according to any one of claims 1 to 12, wherein the composition has a cream form or a paste form.

14. The external-use skin preparation composition according to any one of claims 1 to 13, wherein the composition is applicable as a moisturizer.

15. The external-use skin preparation composition according to any one of claims 1 to 14, wherein the composition is applicable as a lip cosmetic.
